(19) Europäisches Patentamt
European Patent Office
Office européen des brevets

(11) **EP 3 987 943 B1**

(12) **EUROPEAN PATENT SPECIFICATION**

(45) Date of publication and mention
of the grant of the patent:
**18.09.2024 Bulletin 2024/38**

(21) Application number: **21204079.4**

(22) Date of filing: **21.10.2021**

(51) International Patent Classification (IPC):
*A23L 29/10* (2016.01)     *A23L 2/52* (2006.01)
*A23L 33/105* (2016.01)     *A61K 31/352* (2006.01)

(52) Cooperative Patent Classification (CPC):
**A23L 29/10; A23L 2/52; A23L 33/105; A61K 31/05**

(54) **CANNABINOID EMULSIONS**

CANNABINOID-EMULSIONEN

ÉMULSIONS DE CANNABINOÏDES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:   **21.10.2020   US 202063094520 P**

(43) Date of publication of application:
**27.04.2022   Bulletin 2022/17**

(73) Proprietor: **Corn Products Development, Inc.
Westchester, IL 60154 (US)**

(72) Inventors:
• **HOLTHAUS, Derek
Bridgewater, NJ 08807 (US)**
• **LUPITSKYY, Robert
Bridgewater, NJ 08807 (US)**
• **MAGNESS, Scott
Bridgewater, NJ 08807 (US)**

(74) Representative: **Novagraaf Group
Chemin de l'Echo 3
1213 Onex / Geneva (CH)**

(56) References cited:
**WO-A1-2020/024011     WO-A1-2020/044118
WO-A1-2020/123407     US-A1- 2020 054 702
US-A1- 2020 170 944**

**Description**

The technology disclosed in this specification pertains to emulsions comprising a cannabinoid and gum arabic, methods for producing said emulsions and uses of said emulsions.

Cannabinoids are compounds found in cannabis. The best-known cannabinoids are tetrahydrocannabinol (THC) and cannabidiol (CBD). Other cannabinoids include cannabigerol (CBG), cannabichromene (CBC), cannabinol (CBN), cannabielsoin (CBE), *iso*-tetrahydrocannabinol (iso-THC), cannabicyclol (CBL), cannabicitran (CBT), cannabivarin (CBV), tetrahydrocannabivarin (THCV), THCP (tetrahydrocannabiphorol), cannabidivarin (CBDV), cannabichromevarin (CBCV), cannabigerovarin (CBGV), cannabigerol monomethyl ether (CBGM), tetrahydrocannabinolic acid (THCA), cannabidiolic acid (CBDA) and related compounds. Furthermore, cannabinoid compounds may include synthetic cannabinoids. These are generally molecules which are based on the structure of herbal cannabinoids.

Cannabidiol (CBD) oil extracted from hemp and marijuana (Cannabis sativa) is of interest due to its perceived health benefits which range from pain relief to anxiety suppression and beyond. Therefore, there is increased interest in incorporating CBD into foodstuffs to provide the aforementioned health benefits to consumers in an easily deliverable form (foodstuff). However, there are several key challenges associated with adding CBD oils into foodstuffs. First, CBD oils are not water soluble and thus cannot be homogenously incorporated into foodstuffs which are primarily water-based (e.g. beverages). In the case of a beverage, if CBD oil were added into an existing formulation, the oil would float to the top and not evenly distributed throughout the beverage. This creates a significant challenge for dosing and quantification of CBD, as the top portion of the beverage would contain the entirety of the CBD while the rest of the beverage would not contain any. Also, CBD oil generally exhibits low oral bioavailability, as the digestive enzymes and other biological processes can only partially (and slowly) digest CBD oil and transport the CBD to the bloodstream. CBD also is very slow to reach the bloodstream upon oral administration, and thus there is a significant need to speed up the delivery.

The class cannabinoid further comprises other compounds which exhibit various effects. THC is for example the cannabinoid which is the primary psychoactive compound in cannabis.

Gum arabic is a known emulsifier used in a wide variety of foods.

Gum arabic may be from Acacia Senegal or Acacia Seyal. Gum arabic from Acacia Senegal is most commonly used for emulsions.

When using emulsions comprising cannabinoid in the disperse phase, it is believed that a smaller droplet size, and thus a larger surface area, may increase the digestive enzyme function and therefore increases oral bioavailability and time to onset, as well as reduces the required dosing of the cannabinoid to achieve a desired result.

In view of the above, there is a need for an improved emulsion comprising a cannabinoid enabling to combine a relatively small particle size of the disperse oil phase with a relatively high oil load.

Furthermore, there is a need for an improved emulsion comprising a cannabinoid wherein a relatively small particle size of the disperse oil phase may be obtained without requiring polysorbate or any co-surfactant.

US2020/0054702 describes compositions and methods of forming a particulate material derived from a cannabis plant. The method includes introducing a component including at least one of: (i) a cannabinoid, and (ii) a terpene, to a polymer to produce a polymeric mixture. The component is dispersed in the polymeric mixture, which is then at least partially dehydrated to encapsulate the component within a polymeric material derived from the polymer. The polymeric material is water soluble. The dehydrated polymeric mixture is processed to form particulates comprising the component encapsulated within shells formed from the polymeric material.

US2020/0170944 describes water-soluble formulations including cannabinoids or a cannabis-derived compound for use in beverages and foods, methods of preparing the formulations, and methods of preparing beverages and foods including the formulations.

The invention provides an emulsion comprising (i) a continuous aqueous phase, (ii) an disperse oil phase comprising a cannabinoid, and (iii) an emulsifier which is gum arabic, wherein the weight ratio of gum arabic to said disperse oil phase is $\geq$ 3:1, and wherein the weight fraction of the disperse oil phase in the emulsion is from 1 to 20 wt.% based on the weight of the emulsion.

The invention further provides a beverage comprising the emulsion according to the invention.

The invention further provides a method of preparing a beverage, said method comprising incorporating and/or admixing the emulsion according to the invention into said beverage. The invention further pertains to a beverage obtainable by this method.

The invention further provides an emulsion or beverage as described in this specification, for use as a medicament.

As will be understood by the skilled person, the emulsion is an oil-in-water emulsion, wherein oil phase droplets are dispersed within the aqueous continuous phase.

According to the invention, the weight ratio of gum arabic to said disperse oil phase is $\geq$ 3:1. The skilled person will understand that as used herein the weight of the oil phase refers to the sum weight of the components present in the disperse oil phase, excluding emulsifiers present in the emulsion.

In any embodiment described in this specification, the weight ratio of gum arabic to said disperse oil phase is ≥ 4:1. It has been found that an increased weight ratio of gum arabic to the disperse oil phase has the advantage of enabling a larger weight percentage of the disperse oil phase in the emulsion. Furthermore, it has been found that an increased weight ratio of gum arabic to the disperse oil phase has the advantage of enabling a smaller median particle size (d50) without requiring a co-surfactant such as polysorbate.

There is no specific upper limit for the weight ratio of gum arabic to the disperse oil phase. The weight ratio of gum arabic to the disperse oil phase may for instance be ≤ 6:1, or ≤ 5:1.

The weight fraction of the disperse oil phase in the emulsion according to the invention is from 1 to 20 wt.% based on the weight of the emulsion, or from 2 to 15 wt.% based on the weight of the emulsion.

The disperse oil phase may have any suitable particle size. In any embodiment described in this specification, the disperse oil phase has a median particle size (d50) of 500 nm or less, or from 100 to 400 nm, more or from 150 to 300 nm. As used herein the median particle size (d50) is determined by a Malvern apparatus as described in the section "measurement methods"

In any embodiment described in this specification, the weight fraction of the disperse oil phase in the emulsion is from 2 to 8 wt.% based on the weight of the emulsion, more or from 3 to 6 wt.% based on the weight of the emulsion. Applying a weight fraction within these ranges was found to facilitate reducing the particle size of the disperse phase. In any embodiment described in this specification, a weight fraction of the disperse oil phase within these ranges is combined with the weight ratio of gum arabic to the disperse oil phase of ≥ 3:1 or even more or ≥ 4:1. This is found to enable an even further reduction in particle size of the disperse phase, such as a median particle size (d50) of 250 nm or less, or from 100 to 200 nm, or from 150 to 200 nm.

The disperse oil phase may comprise any suitable cannabinoid. In any embodiment, the cannabinoid is selected from the group consisting of tetrahydrocannabinol (THC) and cannabidiol (CBD). In any embodiment the cannabinoid may also be THCA (tetrahydrocannabinolic acid), CBD (cannabidiol), CBDA (cannabidiolic acid), CBN (cannabinol), CBG (cannabigerol), CBC (cannabichromene), CBL (cannabicyclol), CBV (cannabivarin), THCV (tetrahydrocannabivarin), THCP (tetrahydrocannabiphorol), CBDV (cannabidivarin), CBCV (cannabichromevarin), CBGV (cannabigerovarin), CBGM (cannabigerol monomethyl ether), CBE (cannabielsoin), or CBT (cannabicitran).

The disperse oil phase may comprise a vegetable oil. The cannabinoid may be admixed with and/or dissolved in the vegetable oil. The vegetable oil may be any triglyceride oil extracted from seeds. Any suitable vegetable oil may be used, for instance a vegetable oil selected from the group consisting of medium chain triglyceride (MCT) oil, coconut oil, corn oil, cottonseed oil, olive oil, palm oil, peanut oil, rapeseed oil, safflower oil, sesame oil, soybean oil, sunflower oil, and canola oil. Generally, a vegetable oil has a density below that of water, hence below 1.0 g/ml.

It was found that the presence of a vegetable oil as disclosed hereinabove facilitates obtaining a stable emulsion in the event the cannabinoid has a low density. Without wishing to be bound by any scientific theory, it is believed that a vegetable oil having a density between the density of a cannabinoid having a low density and the density of water may assist to minimize the density difference between the disperse and continuous phase, thereby further enhancing the stability of the emulsion.

Based on the teaching provided herein, the skilled person is able to determine suitable ratios between the cannabinoid and the vegetable oil. The weight ratio of the cannabinoid and the vegetable oil may be between 1:0.1 and 1:9, for instance between 1:3 and 3:1. As used herein the weight of the cannabinoid refers to the sum weight of all cannabinoids which may be present in the disperse oil phase.

Any suitable gum arabic may be used. The gum arabic may be gum arabic from *Acacia Senegal* or from *Acacia Seyal.* In any embodiment described in this specification , the gum arabic is gum arabic from *Acacia Senegal.*

It is possible to use gum arabic having an increased molecular weight, such as for instance gum arabic as disclosed in EP-A-1 611 159, EP-A-1 666 502 or gum arabic as disclosed in WO2021/041005. However, this is not necessary.

The emulsion may comprise further emulsifiers in addition to gum arabic. However, this is not necessary. In any embodiment described in this specification, gum arabic is the sole emulsifier in the emulsion. It was found that the presence of non-natural emulsifier such as polysorbate may negatively impact the bioavailability of the cannabinoid. In any embodiment described in this specification the emulsion does not comprise a polysorbate.

The emulsion may optionally contain any suitable optional additive, for instance a preservative. Exemplary additives which may be present in the emulsion include an acid, or an organic acid, for instance citric acid and/or ascorbic acid, potassium sorbate and/or sodium benzoate.

The emulsion can be prepared using methods known in the art. In any embodiment may be prepared by a process comprising:

    i. providing an aqueous phase comprising water and an emulsifier which is gum arabic;
    ii. providing an oil phase comprising an oil and a cannabinoid extract;
    iii. mixing said aqueous phase and said oil phase to create a pre-emulsion; and

iv. homogenizing said pre-emulsion to obtain the emulsion.

In any embodiment described in this specification, the said homogenizing comprises high pressure homogenization at a pressure of at least 130 bar, or between 240 and 2100 bar. In any embodiment described in this specification, the homogenizing is affected in using a microfluidizer. In any embodiment described in this specification, homogenizing is affected using at least 2 passes.

The technology disclosed in this specification can be better understood with reference to the following examples, which are not intended to limit the full scope of the invention.

## Measurement methods

*Turbiscan Stability Index (TSI)*

TSI is a parameter developed specially for formulators to rapidly compare and characterize the physical stability of various formulations and is measured using a Turbiscan Lab Expert (Formulaction) and software TurbiSoft-2.0.0.19. Regarding any embodiment described in this specification, TSI is used to monitor the physical stability of the nanoemulsion concentrate. Any destabilization phenomenon that occurs in a sample will have an impact on the backscattering signal intensities over time. The formulation with the largest change in backscattering intensity is the least stable and has the highest TSI. The calculation of TSI is as follows:

$$TSI = \frac{\sum_h |scan_i(h) - scan_{i-1}(h)|}{H}$$

where the TSI calculation sums up the evolution of backscattered light at all measured position (h), based on a scan-to-scan difference, over total sample height (H).

Turbiscan vials (Formulaction) are filled 4 cm high with each emulsion concentrate and are measured for backscattering several times over a period of 21 days. At day 21, the TSI (Global) is recorded and the emulsion concentrates can be compared against each other for stability against destabilization phenomenon. Larger TSI values correspond to less stable emulsion concentrates.

*Emulsifying Ability Median*

The median particle size (d50), as well as d10, d90, d[4,3] were measured using a particle size analyzer (Manufacturer: Malvern; Model: Mastersizer 2000).

## Examples

Examples 1-6, Reference experiment A

All examples and comparative experiments described herein involved the use of gum arabic from Acacia *Senegal* (TIC Pretested® Gum Arabic Spray Dry Powder).

Nanoemulsions comprising gum arabic as the emulsifier and CBD isolate powder ((>98% purity) purchased from Treehouse Biotech (Longmont, CO) as the cannabinoid source were prepared according to the following formulations (Table 1). All percentages are given in wt.%.

| TABLE 1 | | | | | | | | | |
|---|---|---|---|---|---|---|---|---|---|
| Example | Gum arabic (%) | MCT Oil (%) | CBD Isolate (%) | Citric acid (%) | Ascorbic acid (%) | Potassium Sorbate (%) | Sodium Benzoate (%) | Water (%) | Ratio Gum arabic : oil phase |
| 1 | 20.0 | 3.0 | 2.0 | 0.2 | 0.2 | 0.1 | 0.1 | 74.4 | 5:1 |
| 2 | 25.0 | 3.0 | 2.0 | 0.2 | 0.2 | 0.1 | 0.1 | 69.4 | 5:1 |

(continued)

| TABLE 1 | | | | | | | | | |
|---------|---------------|---------------|---------------------|-------------------|--------------------|--------------------------|-------------------------|-----------|----------------------------------|
| Example | Gum arabic (%) | MCT Oil (%) | CBD Isolate (%) | Citric acid (%) | Ascorbic acid (%) | Potassium Sorbate (%) | Sodium Benzoate (%) | Water (%) | Ratio Gum arabic : oil phase |
| 3 | 20.0 | 6.0 | 4.0 | 0.2 | 0.2 | 0.1 | 0.1 | 69.4 | 2:1 |
| 4 | 25.0 | 6.0 | 4.0 | 0.2 | 0.2 | 0.1 | 0.1 | 64.4 | 2.5:1 |
| 5 (ref) | 20.0 | 7.2 | 4.8 | 0.2 | 0.2 | 0.1 | 0.1 | 67.4 | 1.67:1 |
| 6 (ref) | 20.0 | 7.2 | 4.8 | 0.4 | - | - | 0.05 | 67.55 | 1.67:1 |
| A (ref) | 10.0 | 12.0 | 8.0 | 0.2 | 0.2 | 0.1 | 0.1 | 69.4 | 0.5:1 |

Citric acid, ascorbic acid, sodium benzoate and potassium sorbate were dissolved in room temperature deionized water via overhead mixing for 5 minutes. The gum arabic was added to the solution and allowed to mix for 30 minutes. Simultaneously in a separate beaker, the MCT (medium chain triglyceride) oil was heated on a hot plate to 65 °C. The CBD isolate powder was added to the MCT oil and mixed (via magnetic stirrer bar) until fully dissolved. The CBD oil solution was allowed to cool to room temperature.

A pre-emulsion was made by adding the oil phase into the aqueous phase under high shear mixing conditions (10,000 rpm) for 2 minutes in a homogenizer (Manufacturer: Ross, Model: HSM-LCI-T).

The pre-emulsion was further processed using a Microfluidizer (Microfluidics, Model: M-110EH). The interaction chamber used was the F12Y-H30Z. The mixtures were processed at a pressure of 10000 PSI, or 689.48 bar.

The particle size of the emulsion is immediately tested using a laser diffraction particle size analyzer (Manufacturer: Malvern Mastersizer 2000) where the median particle size (d50), as well as d10, d90, and d[4,3] are recorded. For emulsions with a median particle size (d50) under 100 nm, particle size measurements were taken by dynamic light scattering (DLS) using a Malvern Zetasizer Nano-S and reported as Z-average particle size. Table 2 shows the particle sizes taken after various passes.

The emulsions obtained in examples 1 and 2 have were found to have a median particle size (d50) of below 200 nm. The emulsions obtained in examples 3 to 6 were found to have a median particle size (d50) below 400 nm for emulsions wherein the weight fraction of the disperse phase was as high as 12 wt.%.

| TABLE 2 | | | | | |
|---------|------|----------|----------|----------|------------|
| Example | Pass | d10 (nm) | d50 (nm) | d90 (nm) | d[4,3] (nm) |
| 1 | 1 | 151.85 | 235.91 | 349.18 | 246.11 |
| | 3 | 106.86 | 172.19 | 255.62 | 177.95 |
| | 5 | 108.21 | 175.55 | 262.26 | 181.63 |
| 2 | 1 | 119.54 | 198.88 | 302.51 | 206.44 |
| | 3 | 100.5 | 158.88 | 236.09 | 164.32 |
| | 5 | 98.78 | 156.51 | 232.65 | 161.99 |
| 3 | 1 | 144.96 | 247.24 | 375.06 | 256.15 |
| | 3 | 186.15 | 296.68 | 422.28 | 301.51 |
| | 5 | 227.7 | 349.33 | 486.59 | 354.14 |
| 4 | 1 | 152.65 | 255.57 | 377.67 | 262.04 |
| | 2 | 190.59 | 281.43 | 387.54 | 287.07 |
| | 3 | 135.3 | 226.18 | 335.35 | 232.02 |

(continued)

| TABLE 2 | | | | | |
|---|---|---|---|---|---|
| Example | Pass | d10 (nm) | d50 (nm) | d90 (nm) | d[4,3] (nm) |
| 5 | 1 | 188.33 | 305.92 | 462.69 | 319.55 |
| | 2 | 201.77 | 326.5 | 478.85 | 335.09 |
| | 3 | 238.02 | 378.62 | 539.93 | 385.66 |
| 6 | 2 | 229.5 | 372.11 | 557.76 | 385.48 |
| A (Ref) | 1 | 558.5 | 983.43 | 1352.70 | 975.42 |
| | 2 | 676.56 | 993.13 | 1307.28 | 991.22 |
| | 3 | 687.69 | 994.09 | 1305.77 | 994.66 |

Examples 1.a-6.a, Reference experiment A.a

Beverage stability was evaluated by diluting the nanoemulsions, obtained after the final pass, to a CBD content of 25 mg per 355 g of beverage, such as according to Table 3. The citric acid and sodium benzoate are added to room temperature deionized water and mixed via magnetic stir bar for 5 minutes. The CBD nanoemulsion is added to the solution and lightly mixed. A 12 oz bottle is filled with the solution and capped. The bottle is store horizontally at room temperature without manipulation for 21 days. After 21 days, the beverage is visually examined without manipulation for the presence of a white ring at the top of the beverage (creaming of the CBD emulsion). The beverage can also be examined for sedimentation.

| TABLE 3 | |
|---|---|
| Ingredient | % (w/w) |
| Emulsion (2% CBD isolate; 4%; 4.8%; 8%) | 0.35; 0.7; 0.84; 1.4 |
| Sodium Benzoate | 0.1 |
| Citric acid | 0.3 |
| Water | Balance |

Table 4 provides the beverage stability and TSI of the emulsions.

| TABLE 4 | | |
|---|---|---|
| Example | Beverage Stability | TSI (Global) |
| 1.a | Stable | 0.9 |
| 2.a | Stable | 1.3 |
| 3.a | Stable | 0.8 |
| 4.a | Stable | 0.5 |
| 5.a | Stable | 0.9 |
| 6.a | Stable | 0.8 |
| A.a | Not Stable | 2.7 |

Reference experiment B

A nanoemulsion using polysorbate 80 as the emulsifier was prepared according to Table 5. The polysorbate nanoemulsion was processed at a pressure of 30000 PSI. The particle size measurement was taken by dynamic light scattering (DLS) using a Malvern Zetasizer Nano-S and reported as Z-average particle size. The results have been presented in Table 6.

| TABLE 5 | | | | | | | | |
|---|---|---|---|---|---|---|---|---|
| Experiment B | Polysorbate 80 (%) | MCT Oil (%) | CBD Isolate (%) | Citric Acid (%) | Ascorbic Acid (%) | Potassium Sorbate (%) | Sodium Benzoate (%) | Water (%) |
| | 25.0 | 7.5 | 5.0 | 0.2 | 0.2 | 0.1 | 0.1 | 61.9 |

| TABLE 6 | | | | | | | | |
|---|---|---|---|---|---|---|---|---|
| Experiment B | Pass | d10 (nm) | d50 (nm) | d90 (nm) | Mean (nm) | Beverage Stability | TSI (Global) | |
| | 1 | 84.44 | 133.34 | 199.07 | 138.59 | - | - | |
| | 3 | - | - | - | 42.24 (Z-avg) | - | - | |
| | 4 | - | - | - | 39.78 (Z-avg) | Stable | 24.5 | |

Bioavailability

The bioavailability of the emulsions B, 2 and 5 was determined, and compared to single intravenous cannabidiol (CBD solid) and non-emulsified oil (CBD in MCT oil).

Plasma pharmacokinetics following single intravenous cannabidiol (CBD solid) or oral administration (4 emulsions + 1 non-emulsified oil) was investigated in male Sprague-Dawley rats. Rats were used for this study because they are an accepted model for characterization of pharmacokinetics of formulations being developed for humans. Twelve (12) single-catheterized rats (275-300 g, jugular vein catheter) were obtained from Envigo and divided into 6 groups of 2 animals each. Rats were acclimated for 5 days; the temperature was controlled from 68-79°F, the humidity was controlled from 20% to 70% and the light source was fluorescent lamps with a light/dark cycle of 12/12 hours on/off. No concurrent medication was administered during the study, and all rats had access (ad-libitum) to Tekland Rodent Chow 2018 (Envigo) and tap water throughout the live phase. All animals were randomly placed. Post acclimation, all animals received a single IV (tail vein injection) or oral treatment of one of the nanoemulsions, a non-emulsified oil, or a positive control based on Table 7. Group 1 animals received a single IV injection while Groups 2-6 received a single oral administration. The non-emulsified oil is CBD isolate dissolved in MCT oil. WFI = sterile water for injection. Dosing and dose volume are in mg/mg (body weight) and mL/kg (body weight) respectively.

| TABLE 7 | | | | |
|---|---|---|---|---|
| Emulsion/product | Vehicle | CBD Dose (mg/kg) | Dose Volume (mL/kg) | Route |
| CBD solid | 20% DMSO + WFI | 2 | 5 | IV |
| Non-emulsified oil | MCT Oil | 10 | 10 | PO |
| B | WFI | 10 | 10 | PO |
| 2 | WFI | 10 | 10 | PO |
| 5 | WFI | 10 | 10 | PO |

The CBD concentration in blood plasma samples obtained at various collection times (15, 30, 60, 120, 180, 240, 480 minutes and 24 hr) was determined. The results are reported in Table 8 below.

| TABLE 8 | | |
|---|---|---|
| Sample | Time (min) | Concentration (ng/mL) |
| CBD Isolate | Pre-Dose | ND |
| | 5 | 1560.0 |
| | 30 | 481.0 |
| | 60 | 341.0 |
| | 120 | 178.0 |
| | 180 | 89.2 |
| | 240 | 44.2 |
| | 480 | 10.6 |
| | 24 hr | 5.2 |
| Non-emulsified oil | Pre-Dose | ND |
| | 5 | ND |
| | 30 | ND |
| | 60 | ND |
| | 120 | BLQ |
| | 180 | BLQ |
| | 240 | 6.6 |
| | 480 | 13.6 |
| | 24 hr | BLQ |
| B | Pre-Dose | ND |
| | 5 | 86.2 |
| | 30 | 185.0 |
| | 60 | 310.0 |
| | 120 | 131.0 |
| | 180 | 67.7 |
| | 240 | 45.1 |
| | 480 | 21.6 |
| | 24 hr | 5.4 |
| 2 | Pre-Dose | ND |
| | 5 | 107.0 |
| | 30 | 583.0 |
| | 60 | 262.0 |
| | 120 | 103.0 |
| | 180 | 85.8 |
| | 240 | 66.9 |
| | 480 | 35.5 |
| | 24 hr | 5.0 |

(continued)

| TABLE 8 | | |
|---|---|---|
| Sample | Time (min) | Concentration (ng/mL) |
| 5 | Pre-Dose | ND |
| | 5 | 87.5 |
| | 30 | 294.0 |
| | 60 | 270.0 |
| | 120 | 142.0 |
| | 180 | 88.2 |
| | 240 | 71.7 |
| | 480 | 48.5 |
| | 24 hr | BLQ |

The following pharmacokinetic parameters were determined: Bioavailability absolute (Fabs), Bioavailability relative to non-emulsified (Frel), the time at maximum concentration, the maximum concentration, and half-life ($T_{1/2}$). See Table 9.

| TABLE 9 | | | | | | | |
|---|---|---|---|---|---|---|---|
| Sample | $F_{abs}$ | $F_{rel}$ | $T_{max}$ | $C_{max}$ | $T_{1/2}$ | AUClast (ng*h/mL) | $AUC_{inf}$ (ng*h/mL) |
| CBD solid | | | 0.25 | 1560 | N.D. | 1984 | 2013 |
| Non-emulsified oil | 2.04% | 100% | 8.0 | 13.6 | N.D. | 202 | 276 |
| B | 9.01% | 443% | 1.0 | 310 | 6.89 | 894 | 948 |
| 2 | 12.0% | 589% | 0.5 | 583 | 5.43 | 1190 | 1230 |
| 3 | 8.48% | 416% | 0.5 | 294 | 6.11 | 841 | 1270 |

It can thus be observed that the nanoemulsions achieved maximum concentration faster compared to the polysorbate and non-emulsified oil. They further achieved much higher bioavailability than the emulsified oil.

## Claims

1. An emulsion comprising (i) a continuous aqueous phase, (ii) an disperse oil phase comprising a cannabinoid, and (iii) an emulsifier which is gum arabic, wherein the weight ratio of gum arabic to said disperse oil phase is ≥ 3:1, and wherein the weight fraction of the disperse oil phase in the emulsion is from 1 to 20 wt.% based on the weight of the emulsion, or from 2 to 15 wt.% based on the weight of the emulsion.

2. The emulsion according to claim 1, wherein the weight ratio of gum arabic to said disperse oil phase is ≥ 4:1 to ≤ 6:1, or ≤ 5:1.

3. The emulsion according to any preceding claim, wherein the weight fraction of the disperse oil phase in the emulsion is from 2 to 8 wt.% based on the weight of the emulsion, or from 3 to 6 wt.% based on the weight of the emulsion and wherein said disperse oil phase has a median particle size (d50) of from 100 to 200 nm, more or from 150 to 200 nm.

4. The emulsion according to any preceding claim, wherein said cannabinoid is

   a) one or more of tetrahydrocannabinol (THC) and cannabidiol (CBD) or is
   b) selected from the group consisting of tetrahydrocannabinol (THC) and cannabidiol (CBD) cannabigerol (CBG), cannabichromene (CBC), cannabinol (CBN), cannabielsoin (CBE), *iso*-tetrahydrocannabinol (iso-THC), cannabicyclol (CBL), cannabicitran (CBT), cannabivarin (CBV), tetrahydrocannabivarin (THCV), THCP (tetrahydrocannabiphorol), cannabidivarin (CBDV), cannabichromevarin (CBCV), cannabigerovarin (CBGV), cannabigerol monomethyl ether (CBGM), tetrahydrocannabinolic acid (THCA), cannabidiolic acid (CBDA) or mixtures thereof.

5. The emulsion according to any preceding claim, wherein the oil is a vegetable oil and a weight ratio of said cannabinoid to vegetable oil is between 1:0.1 and 1:9.

6. A beverage comprising the emulsion according to any preceding claim.

7. A method of preparing a beverage, said method comprising incorporating and/or admixing the emulsion according to any preceding claim into said beverage.

8. Beverage obtainable by the method according to claim 7.

9. Emulsion or beverage according to any preceding claim, for use as a medicament.

10. Method for preparing an emulsion according to any one of claims 1 to 5, said method comprising:

    i. providing an aqueous phase comprising water and an emulsifier which is gum arabic,
    ii. providing an oil phase comprising an oil and a cannabinoid extract,
    iii. mixing said aqueous phase and said oil phase to create a pre-emulsion; and
    iv. homogenizing said pre-emulsion to obtain the emulsion.

11. The method of claim 10 wherein the homogenizing is affected using at least 2 passes.

**Patentansprüche**

1. Emulsion, umfassend (i) eine kontinuierliche wässrige Phase, (ii) eine disperse Ölphase, umfassend ein Cannabinoid, und (iii) einen Emulgator, bei dem es sich um Gummi arabicum handelt, wobei das Gewichtsverhältnis von Gummi arabicum zu der dispersen Ölphase ≥ 3:1 ist und wobei der Gewichtsanteil der dispersen Ölphase in der Emulsion von zu 1 bis 20 Gew.-%, bezogen auf das Gewicht der Emulsion, oder von zu 2 bis 15 Gew.-%, bezogen auf das Gewicht der Emulsion, beträgt.

2. Emulsion nach Anspruch 1, wobei das Gewichtsverhältnis von Gummi arabicum zu der dispersen Ölphase ≥ 4:1 bis ≤ 6:1 oder ≤ 5:1 beträgt.

3. Emulsion nach einem der vorstehenden Ansprüche, wobei der Gewichtsanteil der dispersen Ölphase in der Emulsion von zu 2 bis 8 Gew.-%, bezogen auf das Gewicht der Emulsion, oder von zu 3 bis 6 Gew.-%, bezogen auf das Gewicht der Emulsion, beträgt und wobei die disperse Ölphase eine mittlere Partikelgröße (d50) von 100 bis 200 nm, mehr oder von 150 bis 200 nm aufweist.

4. Emulsion nach einem der vorstehenden Ansprüche, wobei das Cannabinoid ist

    a) eines oder mehrere von Tetrahydrocannabinol (THC) und Cannabidiol (CBD) oder es ist
    b) ausgewählt aus der Gruppe bestehend aus Tetrahydrocannabinol (THC) und Cannabidiol (CBD), Cannabigerol (CBG), Cannabichromen (CBC), Cannabinol (CBN), Cannabielsoin (CBE), Iso-Tetrahydrocannabinol (*Iso*-THC), Cannabicyclol (CBL), Cannabicitran (CBT), Cannabivarin (CBV), Tetrahydrocannabivarin (THCV), THCP (Tetrahydrocannabiphorol), Cannabidivarin (CBDV), Cannabichromevarin (CBCV), Cannabigerovarin (CBGV), Cannabigerolmonomethylether (CBGM), Tetrahydrocannabinolsäure (THCA), Cannabidiolsäure (CBDA) oder Mischungen davon.

5. Emulsion nach einem der vorstehenden Ansprüche, wobei das Öl ein Pflanzenöl ist und ein Gewichtsverhältnis des Cannabinoids zu dem Pflanzenöl zwischen 1:0,1 und 1:9 liegt.

6. Getränk, umfassend die Emulsion nach einem der vorstehenden Ansprüche.

**7.** Verfahren zum Herstellen eines Getränks, das Verfahren umfassend ein Einarbeiten und/oder Zumischen der Emulsion nach einem der vorstehenden Ansprüche in das Getränk.

**8.** Getränk erhältlich durch das Verfahren nach Anspruch 7.

**9.** Emulsion oder Getränk nach einem der vorstehenden Ansprüche zur Verwendung als Medikament.

**10.** Verfahren zum Herstellen einer Emulsion nach einem der Ansprüche 1 bis 5, das Verfahren umfassend:

i. Bereitstellen einer wässrigen Phase, umfassend Wasser und einen Emulgator, bei dem es sich um Gummi arabicum handelt,
ii. Bereitstellen einer Ölphase, umfassend ein Öl und einen Cannabinoidextrakt,
iii. Mischen der wässrigen Phase und der Ölphase, um eine Voremulsion zu erzeugen; und
iv. Homogenisieren der Voremulsion, um die Emulsion zu erhalten.

**11.** Verfahren nach Anspruch 10, wobei sich die Homogenisierung unter Verwendung von mindestens 2 Durchläufen auswirkt.

## Revendications

**1.** Émulsion comprenant (i) une phase aqueuse continue, (ii) une phase huileuse dispersée comprenant un cannabinoïde, et (iii) un émulsifiant qui est la gomme arabique, dans laquelle le rapport en poids de la gomme arabique à ladite phase huileuse dispersée est ≥ 3:1, et dans laquelle la fraction en poids de la phase huileuse dispersée dans l'émulsion va de 1 à 20 % en poids par rapport au poids de l'émulsion, ou de 2 à 15 % en poids par rapport au poids de l'émulsion.

**2.** Émulsion selon la revendication 1, dans laquelle le rapport en poids de la gomme arabique à ladite phase huileuse dispersée est ≥ 4:1 à ≤ 6:1, ou ≤ 5:1.

**3.** Émulsion selon l'une quelconque revendication précédente, dans laquelle la fraction en poids de la phase huileuse dispersée dans l'émulsion va de 2 à 8 % en poids par rapport au poids de l'émulsion, ou de 3 à 6 % en poids par rapport au poids de l'émulsion et dans laquelle ladite phase huileuse dispersée présente une granulométrie moyenne (d50) allant de 100 à 200 nm, plus précisément de 150 à 200 nm.

**4.** Émulsion selon l'une quelconque revendication précédente, dans laquelle ledit cannabinoïde est

a) un ou plusieurs parmi un tétrahydrocannabinol (THC) et un cannabidiol (CBD) ou est
b) choisi dans le groupe constitué de tétrahydrocannabinol (THC) et de cannabidiol (CBD) cannabigérol (CBG), cannabichromène (CBC), cannabinol (CBN), cannabielsoïne (CBE), *iso*-tétrahydrocannabinol (*iso*-THC), cannabicyclol (CBL), cannabicitran (CBT), cannabivarin (CBV), tétrahydrocannabivarine (THCV), THCP (tétrahydrocannabiphorol), cannabidivarine (CBDV), cannabichromévarine (CBCV), cannabigerovarine (CBGV), éther monométhylique de cannabigérol (CBGM), acide tétrahydrocannabinolique (THCA), acide cannabidiolique (CBDA) ou des mélanges de ceux-ci.

**5.** Émulsion selon l'une quelconque revendication précédente, dans laquelle l'huile est une huile végétale et le rapport en poids dudit cannabinoïde à l'huile végétale est compris entre 1:0,1 et 1:9.

**6.** Boisson comprenant l'émulsion selon l'une quelconque revendication précédente.

**7.** Procédé de préparation d'une boisson, ledit procédé comprenant l'incorporation et/ou le mélange de l'émulsion selon l'une quelconque revendication précédente dans ladite boisson.

**8.** Boisson pouvant être obtenue par le procédé selon la revendication 7.

**9.** Émulsion ou boisson selon l'une quelconque revendication précédente, destinée à être utilisée comme médicament.

**10.** Procédé de préparation d'une émulsion selon l'une quelconque des revendications 1 à 5, ledit procédé comprenant :

i. la fourniture d'une phase aqueuse comprenant de l'eau et un émulsifiant qui est la gomme arabique,
ii. la fourniture d'une phase huileuse comprenant une huile et un extrait de cannabinoïde,
iii. le mélange de ladite phase aqueuse et de ladite phase huileuse pour créer une pré-émulsion ; et
iv. l'homogénéisation de ladite pré-émulsion pour obtenir l'émulsion.

**11.** Procédé selon la revendication 10, dans lequel l'homogénéisation est effectuée à au moins deux reprises.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20200054702 A
- US 20200170944 A
- EP 1611159 A
- EP 1666502 A
- WO 2021041005 A